# EUROPEAN PATENT APPLICATION

(11) **EP 3 501 585 A1**
(43) Date of publication of application: **26.06.2019**
(21) Application number: 18382918.3
(22) Date of filing: 12.12.2018
(51) Int. Cl.: A61M 16/04, A61F 2/20

(54) **SECURING ACCESSORY FOR AN ASSEMBLY FOR TRACHEAL STOMA**

(30) Priority: 22.12.2017 ES 201731457
(71) Applicant: Borja Barrones, Daniel, 29603 Marbella (ES); Fuentes Bernal, Rafael, 29603 Marbella (ES); Ruiz Lopez, Alejandro, 29603 Marbella (ES); 3D Zings Additive Tech, S.L., 29590 Malaga (ES)
(72) Inventor: RUIZ LOPEZ, Alejandro, 29603 Marbella (ES)
(74) Representative: Isern Patentes y Marcas S.L.

(57) **Abstract**

The invention relates to a securing accessory (1) for a tracheal stoma assembly (2), the tracheal stoma assembly comprising a fastening support (3) configured to be fastened in an area close to the tracheal stoma (ET) and a device (4) for tracheal stoma in fluid connection with the tracheal stoma and configured to attach to the fastening support, wherein the securing accessory comprises means (5) of fastening to a user; and anchoring means (6) configured to couple to the fastening support of the assembly for tracheal stoma and to the means of fastening to the user's body; and further configured to house the device for tracheal stoma.

## Description

### TECHNICAL FIELD OF THE INVENTION

The present invention is related to the field of medical devices, in particular, with a support for a device that couples in the stoma of a trachea, after a tracheostomy procedure. More particularly, the present invention relates to a support that aids in securing devices that are usually fastened as an adhesive in the area around the tracheal stoma, ensuring that the integrity of the adhesive bond lasts longer.

### STATE OF THE ART

When there are failures in the respiratory tracts of a person that prevent them from getting enough air due to injuries, disorders or impairments of the nervous or respiratory systems, in order to restore the airway and enable the proper functioning thereof, a surgical procedure called tracheostomy is carried out which consists of creating an opening, known as stoma, in the trachea through an incision made in the neck and inserting a tube or cannula with an inflatable sleeve that occludes the space between the walls of the trachea and the cannula, to facilitate the passage of air towards the lungs. Tracheostomy provides an artificial airway; however, due to the same, the air no longer passes through the upper trachea and the oropharynx, unless the cannula does not have a sleeve, the sleeve is deflated or a speaking valve is used. The lack of airflow through the upper respiratory tracts reduces the tone and pharyngeal and laryngeal sensation and prevents the effective glottic closure, minimising or taking away one's ability to speak.

As a result of the tracheostomy procedure, the inhaled and exhaled air can pass through the tracheal stoma and, if a voice prosthesis has been placed, the stoma can occlude so that the exhaled air is diverted through the voice prothesis towards the pharynx and the mouth, allowing the person to speak. It is desirable for the flow of the exhaled air to be controlled by means of a valve located in the tracheal stoma. In these cases, the valve can be arranged to remain open during breathing, but it can be closed to divert the airflow by means of a small additional increase in the flow of exhaled air.

Multiple devices intended for coupling in the tracheal stoma, such as filters, tracheal humidifiers, respiratory protectors and speaking valves, have been developed in order to provide users with alternative ways to recover some of the eliminated functions when the air does not pass through the nose, among which include restoring the ability to speak. Said devices are used together with a support that keeps them in their place, such that they can be called assembly for tracheal stoma, wherein said support is arranged above the tracheal stoma of a person, and they can be fastened to the skin by means of an adhesive strip, wherein the adhesive is oriented towards the user's skin.

One widely known problem that is related to the fastening of the support for the device for tracheal stoma is the limited duration of the adhesive effect of said support on the skin due to factors such as the irregularity of the skin, sweat, deformations in the area, collapse of the tracheal stoma, pressure of the airflow leaving through the stoma, among others. This limited effect of adhesion makes it so that the support becomes unattached much sooner than expected, such that the device for tracheal stoma, such as a speaking valve, ceases to function properly, and the assembly loses its position with respect to the tracheal stoma, where in many cases the user has to intervene, holding the support to prevent it from prematurely detaching further. This recurring problem means that the users end up discarding or minimally using the devices for tracheal stoma.

Some solutions are currently offered to increase the adhesive duration of the support on the skin by means of products such as special glues which, in addition to being costly, may irritate and affect the skin around the stoma, without properly solving the problem described.

Therefore, it is obvious that there is a need to provide an accessory that helps secure the assembly for tracheal stoma, wherein said accessory increases the time that it remains fastened with respect to the tracheal stoma.

### DESCRIPTION

To overcome the drawback found, the present invention provides a securing accessory for an assembly for tracheal stoma, the assembly for tracheal stoma comprising a fastening support configured to be fastened in an area close to the tracheal stoma and a device for tracheal stoma in fluid connection with the tracheal stoma and configured to attach to the fastening support, wherein the securing accessory comprises means of fastening to a user; and anchoring means configured to couple to the fastening support of the assembly for tracheal stoma and to the means of fastening to the user's body; and further configured to house the device for tracheal stoma.

In alternative embodiments of the securing accessory for an assembly for tracheal stoma, the means of fastening to the user's body comprise an assembly of belts intended to be secured on the user's torso.

In other alternative embodiments of the securing accessory for an assembly for stoma, the anchoring means comprise an elastic plate configured to couple to the fastening support of the assembly for tracheal stoma; and an anchoring plate configured to couple to the elastic plate and that is provided with fastening means configured to couple the assembly of belts to said anchoring plate.

The main advantage achieved with the securing accessory of the present invention is that by externally securing the fastening support, support is provided to the adhesive bond between said fastening support and the user's skin, thereby maintaining the integrity thereof longer, such that the assembly for tracheal stoma keeps the functional position thereof without requiring the user's attention.

### BRIEF DESCRIPTION OF THE FIGURES

The previous advantages and features, in addition to others, shall be understood more fully in light of the following detailed description of exemplary embodiments, with reference to the drawings attached, which must be taken by way of illustration and not limitation, wherein:
- Fig. 1 is a perspective view of a preferred embodiment of the invention wherein the securing accessory is arranged on a user.
- Fig. 2 is a perspective view of a common assembly for tracheal stoma, the assembly comprising a fastening support and a device for tracheal stoma.
- Fig. 3 is a perspective view of the fastening accessory of a preferred embodiment, wherein the user is not shown but the assembly for tracheal stoma is visible.
- Fig. 4 is an exploded perspective view of the anchoring means, wherein the assembly for tracheal stoma is visible.

### DETAILED DESCRIPTION OF AN EXEMPLARY EMBODIMENT

The following detailed description presents a number of specific details by way of example to provide a detailed understanding of the relevant teachings. However, it is clear for a person skilled in the art that the present teachings can be carried out without these details.

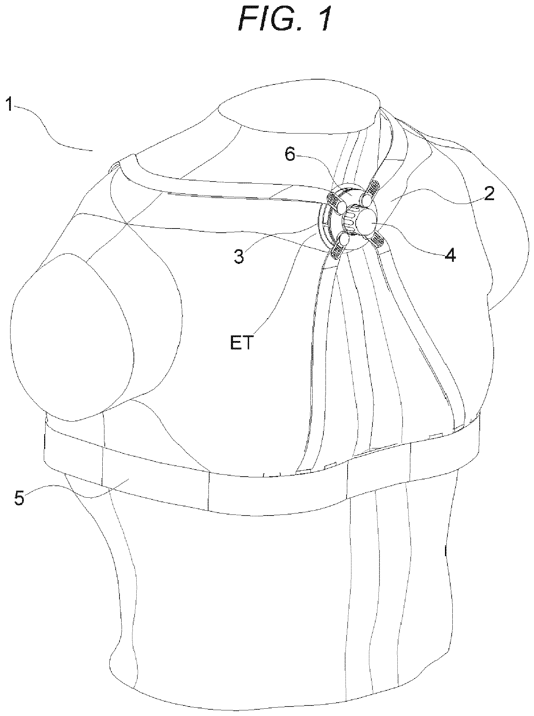

According to a first embodiment, and as can be observed in Figure 1, the invention discloses a securing assembly (1) for an assembly for tracheal stoma (2), the assembly for tracheal stoma (2) comprising a fastening support (3) configured to be fastened in an area close to a tracheal stoma (ET) and a device for tracheal stoma (4) in fluid connection with the tracheal stoma (ET) and configured to attach to the fastening support (3), wherein the securing accessory (1) comprises means of fastening to a user (5) and anchoring means (6) configured to couple to the fastening support (3) of the assembly for tracheal stoma (2) and to the means of fastening to a user (5), wherein the anchoring means (6) are further configured to house the device for tracheal stoma (4).

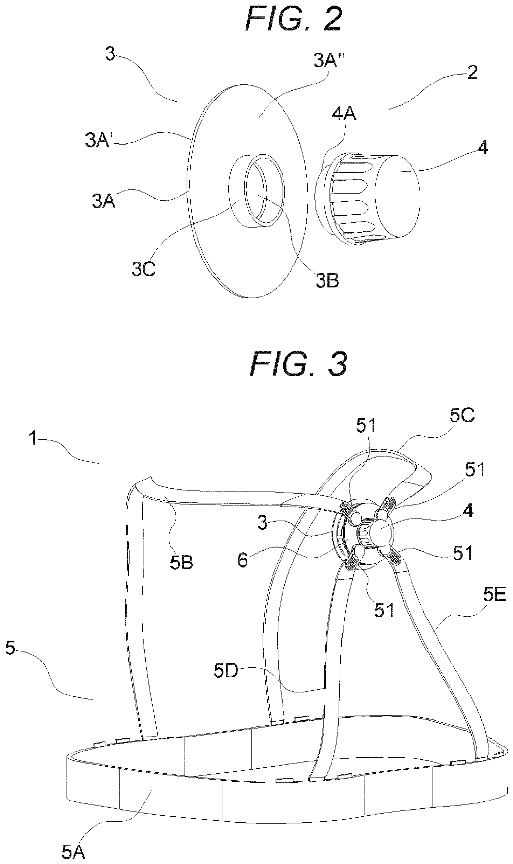

The fastening supports (3) are widely known in the field of medical devices used when tracheostomy procedures have been carried out. As shown in Figure 2, in a typical assembly for tracheal stoma (2), the fastening supports (2) are designed to be secured by means of an adhesive bond to the user's skin and they generally comprise a base plate (3A) which has a hole (3B) that passes through said base plate (3A). The base (3A) comprises an adhesive side (3A') that is oriented towards the user and is configured to adhere to the skin of said user, and an outer side (3A") that is oriented towards the outside. Enclosing the hole (3B) and fastened to the base (3A) is a tubular coupling (3C) for fastening the device for tracheal stoma (4), wherein said tubular coupling (3C) is a tubular-type coupling such as a sleeve or a hollow cylindrical body. When the fastening support (3) is arranged on the user, the base plate (3A) should be fastened in a way that the hole (3B) is aligned with the tracheal stoma (ET) to establish fluid communication between the tubular coupling (3C) and said tracheal stoma (ET). Likewise, the adhesive fastening of the base pate (3A) with the skin should be such that the inhaled and exhaled air only has the tubular coupling (3C) of the fastening device (3) as a flow path. The coupling (3C) generally comprises fastening means (not shown) configured to fasten the device for tracheal stoma (4). Said fastening means can comprise an annular protrusion (not shown) in the inner wall of the tubular coupling (3C). This recess is configured to couple an annular protrusion (not shown) defined in a connection portion (4A) of the device for tracheal stoma (4). Alternatively, the inner wall of the tubular protrusion (3C) can have an annular protrusion (not shown) configured to be coupled to an annular protrusion (not shown) defined in a connection portion (4A) of the device for tracheal stoma (4). Since the fastening supports (3) and the devices for tracheal stoma (4) are devices on which the present invention is applied, the specific configuration of said devices is not relevant provided that they fulfil the following conditions: the base (3A) must be fastened as an adhesive on the user with the tubular coupling (3C) aligned and in fluid communication with the tracheal stoma (ET) and the device for tracheal stoma (4) is coupled in a releasable way in said tubular coupling (3C).

On the other hand, the device for tracheal stoma (4) intended to be coupled in the fastening support (3) can be one of a filter, tracheal humidifier, respiratory protector, speaking valve, etc. In the preferred embodiment, the device for tracheal stoma (4) comprises a speaking valve.

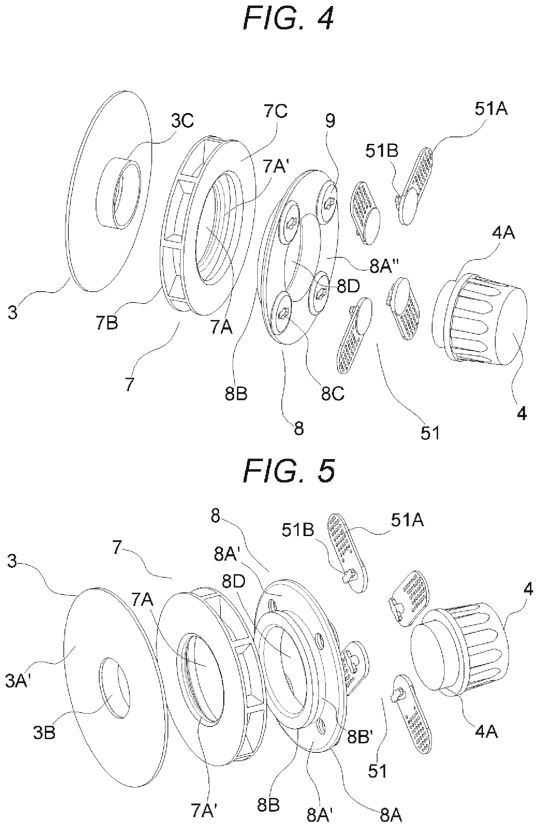

According to that observed in Figure 4, the means of fastening to a user (5) of the securing accessory (1) comprise an assembly of belts intended to be secured on the user's chest. Said assembly of belts is made up of a chest-securing belt (5A) intended to surround and be adjusted to the user's chest, a right shoulder strap (5B), a left shoulder strap (5C), a front right strap (5D) and a front left strap (5E), wherein said straps each comprise a first end configured to be fastened to the chest-securing belt (5A) and a second end configured to couple to the anchoring means (6), each of the straps (5B to 5E) being configured to adjust to the user's chest. Preferably, in each one of said second ends there is a connector (51) configured to be fastened in the anchoring means (6) of the securing accessory (1). As shown in greater detail in Figure 4, the connector (51) in turn comprises a first portion (51A) configured to be secured to the strap and a protruding portion (51B) that, as will be seen below, is configured to couple to an anchoring plate (8) of the anchoring means (6). To provide a feeling of comfort to the user, the assembly of belts are belts of an elastic type made up of textile materials, for example, a first elastic inner material that is coated by a second elastic, resistant outer material, said elastic materials being able to be selected from neoprene, among others. Likewise, each of the straps (5B, 5C, 5D, 5E) as well as the chest-securing belt (5A) have length adjusting means configured to adjust the assembly of belts to the dimensions of the user's body. In the case of the straps (5B, 5C, 5D, 5E), the length adjusting means are carried out in the same way that the straps of a woman's bra are adjusted, which is widely known; meanwhile, for the chest-securing belt, adjusting means similar to that of the straps or a unitary elastic belt that expands in order to couple to the size of the chest can be used. Other ways of adjusting the length of the assembly of belts can be used without deviating from the scope of the invention.

On the other hand, and as can be seen in Figure 4, the anchoring means (6) comprise an elastic plate (7) configured to couple to the fastening support (3) of the assembly for tracheal stoma (2), and an anchoring plate (8) configured to couple to the elastic plate (7) and that is provided with securing means (9) configured to couple the assembly of belts (5) to said anchoring plate (8). The elastic plate (7) comprises a through hole (7A) configured to receive the tubular coupling (3C) and an inner face (7B) configured to rest on the securing support (3) and an outer face (7C) configured to rest on the anchoring plate (8) according to what was observed in Figure 4. In the inner wall of the through hole (7A) there is a protrusion (7A') that as described in subsequent lines is configured to couple to a recess (8B') defined in a tubular portion (8B) of the anchoring plate (8). The main function of the elastic plate (7) is to be used as a damping element between the anchoring plate (8) and the fastening support (3) in order to ensure that the securing given to said fastening support (3) is of the elastic type, such that the user of the fastening accessory (1) can move freely, without fear of the different body positions creating additional stresses on the fastening support (3) through the anchoring plate (8). Given the nature of the movements that a person can make during a journey, it is possible that some postures provoke unexpected stresses or loads on the anchoring plate (8) through the straps (5B, 5C, 5D, 5E); for this reason the elastic plate (7) has been configured to absorb all these possible deformations, maintaining uniform securing pressure of the anchoring plate (8) on the fastening support (3) to thus prolong the integrity of the adhesive bond of the fastening support (3) with the user's skin. Therefore, the fastening accessory (1) of the present invention provides an integral support to the assembly for tracheal stoma (2) formed by the fastening support (3) and the device for tracheal stoma (4). The material for the elastic plastic (7) can be chosen from several different classes of polymers, provided that they fulfil the requirements of low hardness, dimensional stability and absorption of deformations. One example of the material from which the plate can be manufactured is the Dow Corning® EE-3200 polymer from the Dow Corning Corporation company. This should not be understood as limiting but rather a simple application of the material from which the plate can be manufactured, taking into account that other materials are also applicable without deviating from the invention.

In preferred embodiments, the elastic plate (7) is configured as a circular plate, the through hole (7A) being a circular through hole, wherein the protrusion (7A') is an annular protrusion.

As shown in Figures 4 and 5, the anchoring plate (8) comprises an outer plate (8A) and a tubular portion (8B) that extends from the outer plate (8A), wherein the outer dimension of said tubular portion (8B) is configured to tightly couple in the through hole (7A) of the elastic plate (7). Likewise, in said tubular portion (8B) there is a recess (8B') configured to receive the protrusion (7A') of the through hole (7A) of the elastic plate (7). In preferred embodiments, the outer plate (8A) is a circular plate and the tubular portion (8B) is a cylindrical sleeve, wherein the recess (8B') is an annular recess. On the other and, the outer plate (8A) comprises an inner face (8A') from which the tubular portion (8B) projects and wherein the outer face (7C) of the elastic plate (7) rests, and an outer face (8A") wherein fastening means are provided configured to fasten the straps (5B, 5C, 5D, 5E) of the assembly of belts. Said fastening means preferably comprise holes (8C) distributed on the outer face (8A") configured to couple the protruding portion (51B) of the connector (51) arranged in each of the straps (5B, 5C, 5D, 5E). In the preferred embodiment shown in Figures 4 and 5, the fastening means comprise at least 4 holes (8C). Likewise, the anchoring plate comprises a through hole (8D) configured to house the device for tracheal stoma (4) and to enable said device (4) to couple in the fastening support (3). The material from which the anchoring plate (8) is made is preferably selected from a rigid polymer with high mechanical resistance with high dimensional stability, such as for example the PA 2200 polymer of the EOS GmbH company. This should not be understood as limiting, since other materials can be applied without deviating from the scope of the invention.

## Claims

1. A securing assembly (1) for an assembly for tracheal stoma (2), the assembly for tracheal stoma comprising a fastening support (3) configured to be fastened in an area close to the tracheal stoma (ET) and a device for tracheal stoma (4) in fluid connection with the tracheal stoma (ET) and configured to attach to the fastening support (3), the securing accessory (1) being **characterised in that** it comprises:
- means of fastening to a user (5); and
- anchoring means (6) configured to couple to the fastening support (3) of the assembly for tracheal stoma (2) and to the means of fastening to a user (5); and further configured to house the device for tracheal stoma (4).

2. The securing assembly (1) for an assembly for tracheal stoma (2) according to claim 1, wherein the means of fastening to a user (5) are **characterized in that** they comprise:
- an assembly of belts intended to be secured on the user's chest.

3. The securing assembly (1) for an assembly for tracheal stoma (2) according to claim 2, wherein the anchoring means (6) are **characterized in that** they comprise:
- an elastic plate (7) configured to couple to the fastening support (3) of the assembly for tracheal stoma (2); and
- an anchoring plate (8) configured to couple to the elastic plate (7) and that is provided with fastening means configured to couple the assembly of belts to said anchoring plate.
